# EUROPEAN PATENT SPECIFICATION

(11) **EP 2 442 837 B1**
(45) Date of publication and mention of the grant of the patent: **20.08.2014**
(21) Application number: 10724868.4
(22) Date of filing: 18.06.2010
(51) Int. Cl.: A61L 27/36, A61P 7/06

(54) **BONE MARROW EXTRACELLULAR MATRIX EXTRACT AND THERAPEUTIC USE THEREOF**
EXTRAZELLULÄRER KNOCHENMARK-MATRIXEXTRAKT UND SEINE THERAPEUTISCHE VERWENDUNG
EXTRAIT EXTRACELLULAIRE DE MOELLE OSSEUSE ET UTILISATION THÉRAPEUTIQUE DE CELUI-CI

(30) Priority: 18.06.2009 US 218182 P
(43) Date of publication of application: 25.04.2012
(73) Proprietor: MC2 Cell Aps, 2970 Hørsholm (DK)
(72) Inventor: EL-SABBAN, Marwan, DK-5230 Odense M (DK)
(74) Representative: Orsnes, Henrik Egede
(86) International application number: PCT/EP2010/058675
(87) International publication number: WO 2010/146177

(56) References cited:
- WO-A1-97/18842
- WO-A1-2009/080794
- WO-A2-2005/009498
- WO-A2-2007/056547
- EL-SABBAN MARWAN E ET AL: "Xenogenic bone matrix extracts induce osteoblastic differentiation of human bone marrow-derived mesenchymal stem cells." July 2007 (2007-07), REGENERATIVE MEDICINE JUL 2007 LNKD- PUBMED:17635046, VOL. 2, NR. 4, PAGE(S) 383 - 390 , XP002597628 ISSN: 1746-076X the whole document
- MATROSOVA VERA Y ET AL: "Hyaluronic acid facilitates the recovery of Hematopoiesis following 5-fluorouracil administration" 2004, STEM CELLS (MIAMISBURG), VOL. 22, NR. 4, PAGE(S) 544-555 , XP002597629 ISSN: 1066-5099 * abstract
- BAZARBACHI A ET AL: "XENOGENIC CARTILAGE MATRIX EXTRACTS SPECIFICALLY INDUCE CHONDROCYTIC DIFFERENTIATION OF HUMAN BONE MARROW DERIVED MESENCHYMAL STEM CELLS" June 2008 (2008-06), HAEMATOLOGICA-THE HEMATOLOGY JOURNAL, VOL. 93, NR. SUPPL. 1, PAGE(S) 505-506 URL , 13TH CONGRESS OF THE EUROPEAN-HEMATOLOGY-ASSOCIATION; COPENHAGEN, DENMARK; JUNE 12 -15, 2008 , XP002597630 ISSN: 0390-6078(print) 1592-8721(ele * abstract
- KHURANA SATISH ET AL: "In vitro transdifferentiation of adult hematopoietic stem cells: An alternative source of engraftable hepatocytes" December 2008 (2008-12), JOURNAL OF HEPATOLOGY, VOL. 49, NR. 6, PAGE(S) 998-1007 , XP002597631 ISSN: 0168-8278 the whole document
- DALAKAS EVANGELOS ET AL: "Hematopoietic stem cell trafficking in liver injury" August 2005 (2005-08), FASEB JOURNAL, VOL. 19, NR. 10, PAGE(S) 1225-1231 , XP002597632 ISSN: 0892-6638
- RENTALA ET AL: "MDR1 gene expression enhances long-term engraftibility of cultured bone marrow cells" BIOCHEMICAL AND BIOPHYSICAL RESEARCH COMMUNICATIONS, ACADEMIC PRESS INC. ORLANDO, FL, US LNKD- DOI:10.1016/J.BBRC.2005.07.167, vol. 335, no. 3, 30 September 2005 (2005-09-30), pages 957-964, XP005100413 ISSN: 0006-291X
- SEITA JUN ET AL: "Interleukin-27 directly induces differentiation in hematopoietic stem cells" February 2008 (2008-02), BLOOD, VOL. 111, NR. 4, PAGE(S) 1903-1912 , XP002597633 ISSN: 0006-4971
- WAGNER WOLFGANG ET AL: "The stromal activity of mesenchymal stromal cells" 2008, TRANSFUSION MEDICINE AND HEMOTHERAPY, VOL. 35, NR. 3, PAGE(S) 185-193 , XP002597634 ISSN: 1660-3796

## Description

### BACKGROUND OF THE INVENTION

The present invention relates to the identification and use of hematopoietic stem cells (HSC). More specifically the invention relates to the differentiation autologueous hematopoietic stem cells into myeloid lineages for reintroduction into the patient in addition to undifferentiated stem cells for the purpose of reconstituting both the defence and long-term engraftment.

Bone marrow (BM) transplantation is being increasingly used in humans as an effective therapy for an increasing number of diseases, including malignancies such as leukemias, lymphoma, myeloma and selected solid tumors as well as nonmalignant conditions such as severe aplastic anemias, immunologic deficiencies and inborn errors of metabolism. The objective of BM transplantation is to provide the host with a healthy stem cell population that will differentiate into mature blood cells that replace deficient or pathologic cell lineages. The source of the BM for transplantation may be autologous, syngeneic or allogeneic. Preferred are autologous BM or BM from HLA-matched siblings, but also BM from HLA-nomnatched donors is being used for transplantation.

The only known clinical condition in which complete systemic donor-specific transplantation tolerance occurs is when chimerism is created through bone marrow transplantation. This has been achieved in neonatal and adult animal models as well as in humans by total lymphoid or body irradiation of a recipient followed by bone marrow transplantation with donor cells. The success rate of allogenic bone marrow transplantation is, in large part, dependent on the ability to closely match the "major histocompatability complex" (MHC) of the donor cells with that of the recipient cells to minimize the antigenic differences between the donor and the recipient, thereby reducing the frequency of host-versus-graft responses and "graft-versus host disease" (GVHD). In fact, MHC matching is essential, only a one or two antigen mismatch is acceptable because GVHD is very severe in cases of greater disparities.

Hematopoietic stem cells are rare cells that have been identified in fetal bone marrow, umbilical cord blood, adult bone marrow, and peripheral blood, which are capable of differentiating into each of the myeloerythroid (red blood cells, granulocytes, monocytes), megakaryocyte (platelets) and lymphoid (T-cells, B-cells, and natural killer cells) lineages. In addition these cells are long-lived, and are capable of producing additional stem cells, a process termed self-renewal. Stem cells initially undergo commitment to lineage restricted progenitor cells, which can be assayed by their ability to form colonies in semisolid media. Progenitor cells are restricted in their ability to undergo multi-lineage differentiation and have lost their ability to self-renew. Progenitor cells eventually differentiate and mature into each of the functional elements of the blood. The lifelong maintenance of mature blood cells results from the proliferative activity of a small number of pluripotent hematopoietic stem cells that have a high, but perhaps limited, capacity for self- renewal. In culture, hematopoietic stem cells rapidly commit to differentiated cell types, which irreversibly predominate in the culture. This property, along with their relative scarcity in blood, presents challenges to the creation of long term, stable cultures of pluripotent hematopoietic stem cells.

Accumulating evidence indicates that stem cell homing to the bone marrow is a multistep process. The mechanisms and specific adhesion molecules involved in this process are not fully understood. The PI integrins, very late antigen 4 (VLA-4) and VLA-5, and the P2 integrin lymphocyte function-associated 1 (LFA-1) have been shown to be implicated in the adhesive interactions of both mouse and human progenitor cells with the bone marrow extracellular matrix (ECM), as well as with bone marrow stromal cells (Levesque et al., 1995). VLA-4 plays an especially important role in murine stem cell migration and hematopoiesis in vivo. Murine stem cells lacking P I integrins fail to colonize the fetal liver (Hirsh et al., 1996).

WO2005/009498A2 discloses animal (human) bone marrow extracellular matrix extract and soluble factors, however, not for the purpose of reconstituting both the defence and long-term engraftment in autologous hematopoietic cell transplantation.

EL-SABBAN MARWAN et al (REGENERATIVE MEDICINE, VOL. 2, pp 383-390) disclose the role of hyaluronic acid, a major component of the bone marrow extracellular matrix, in the differentiation of hematopoietic stem cells into progenitor cells (myeloid cells). Meanwhile, there is no disclosure of autologous HSCs transplantation or combining the HSCs and the myeloid cell lineage to form a cellular preparation.

In view of the expanded approach to the treatment of many severe diseases by hematopoietic stem cell treatment, it is highly desirable to understand better the mechanism behind stem cell homing to the bone marrow and repopulation of transplanted hosts in order to obtain stem cells with higher rates of successful and long-tem engraftment.

Two barriers associated with bone marrow transplantation BMT have limited its application to clinical transplantation: (1) graft-versus-host disease (GVHD) and (2) failure of engraftment.

BMT has the potential to treat a number of disorders, including cancer (lymphomas), hemoglobinopathies (sickle cell disease, thalassemia), soluble enzyme deficiencies, and autoimmune disorders. The morbidity and mortality associated with transplantation of unmodified marrow has prevented the widespread application of this approach. Conventional T cell depletion prevents graft versus host disease but is associated with an unacceptably high rate of graft failure. Therefore, there remains a need for an optimization of engraftment procedures.

### SUMMARY OF THE INVENTION

Accordingly, it is an object of the present invention to provide

According to the invention there is provided a cellular preparation for use in autologous hematopoietic cell transplantation in a patient by administering the cellular preparation to the patient for the purpose of reconstituting both the defence and long-term engraftment, wherein said cellular preparation is obtainable by a method involving:
i) removing hematopoietic stem cells from the patient;
ii) optionally expanding the hematopoietic stem cells ex vivo with a growth factor selected from the group consisting of GM-CSF, SF, IL-3, IL- 1, GM-CSF/IL-3 fusion proteins, and combinations thereof to provide a cellular preparation comprising an expanded population of hematopoietic progenitor cells;
iii) contacting a fraction of the hematopoietic stem cells with a human bone marrow extracellular matrix extract and soluble factors to differentiate the cells into a myeloid cell lineage; and
iv) combining the hematopoietic stem cells and the myeloid cell lineage to form the cellular preparation.

The bone marrow extracellular matrix extract may be of human or animal origin, such as equine, canine, porcine, bovine, and ovine sources; or rodent species, such as mouse or rat. Although the aim of the present invention is to produce in vitro an extracellular matrix and soluble factors, which composition reflects the composition in a human being and thus essentially consists of growth factors, minerals etc secreted from human cells, an animal extracellular matrix extract may be used to induce the differentiation and thus stimulate the human stem cells to secrete an extracellular matrix of human origin. As the human stem cells differentiate they will produce an extracellular matrix layer or body, which predominantly contains differentiated human cells along with the extracellular matrix produced by them-selves; thus, if the stem cells have been stimulated to differentiate in an extract of animal origin there will be no fractions of non-human material left in the produced extracellular matrix layer. Since the in vivo composition of the extracellular matrix is achieved when the stem cells are fully differentiated into the cells of the tissue of interest it is often necessary to cultivate the stem cells for generally at least 14 days. In any event the cells are assessed before harvesting the extracellular matrix; as a general rule at least 90% of the cells should be fully differentiated before preparing the extracellular matrix.

### DETAILED DESCRIPTION OF THE INVENTION

Extracellular matrix and soluble factors of the bone marrow tissue can be used in vivo to induce hematopoietic progenitor cells in bone marrow to proliferate and to mobilize such hematopoietic progenitor cells into peripheral blood. Hematopoietic progenitor cells harvested from peripheral blood can be used for hematopoietic rescue therapy of patients treated with cytoreductive agents.

The present invention involves ex vivo treatment of hematopoietic progenitor cells from peripheral blood or bone marrow with growth factors to increase their numbers prior to infusion or transplantation. In addition, growth factors can be used to facilitate engraftment and proliferation of transplanted hematopoietic progenitor cells following transplantation. Hematopoietic reconstitution of a patient undergoing cytoreductive therapy can reduce the incidence of infection and bleeding complications of patients treated with high doses of cytoreductive therapies, such as myelosuppressive cancer chemotherapeutic agents or high doses of radiotherapy.

The development of ex viva culture conditions that facilitate the expansion and differentiation of hematopoietic stem cells (HSCs) would greatly accelerate the clinical implementation of next generation therapeutics including cell transplantation, gene therapy and tissue engineering. In fact, the last few years have shown an increase in the clinical utility of such cells in transplantation therapies. Unfortunately, the establishment of culture conditions capable of consistently and efficiently growing HSCs in vitro has been elusive. Current strategies aimed at expanding HSCs, primarily through growth factor supplementation or stromal cell support, generally result in the expansion of mature progenitors, but are complicated by the losses or moderate expansions of more primitive cells following short-term culture.

These results are somewhat surprising since, in viva, HSCs have been shown to have extensive proliferative potential. Experiments demonstrating that long-term engraftment in mice can be achieved by the progeny of a single murine or human cell, and that the progeny of a single clone can repopulate multiple secondary recipients provide evidence of this potential. Furthermore, serial transplant studies in mice, in which input and output numbers of repopulating stem cells were monitored and quantified at each passage, have convincingly shown that repopulating stem cells are capable of sustained in vivo expansion where theoretical 150-8400 fold expansions have been calculated. Therefore, it is apparent that simple media supplementation is not sufficient to overcome the growth inhibitory effects seen in most in vitro systems.

As used herein, the terms "undifferentiated hematopoietic cell", "undifferentiated cell", "hematopoietic stem cell (HSC)", and "primitive cell" are used interchangeably to describe a pluripotential hematopoietic stem cell that is capable of long term in viva expansion and repopulation when transplanted into a mammal. It has been established that the most primitive cell types express the cell surface antigen CD34, which is a transmembrane glycophosphoprotein thought to play an important role in stem and progenitor cell adhesion in BM. Cell populations expressing CD34 and lacking the CD38 antigen (i.e. CD34+CD38-cells) have been shown to display primitive cell potentials. For example, the majority of SRCs can be found in the CD34+ CD38- cell fractions and not in the CD34+CD38+ populations, which are thought to contain more differentiated cell types. The CD34+CD38-phenotype has also been associated with an enrichment of cells having LTC-IC characteristics. The existence of murine and human CD34- HSCs that are capable of long-term multilineage repopulation illustrates that the CD34 antigen may itself be regulated independently of HSC potential and that CD34 expression itself is not a requisite HSC marker. Primitive cells have also been identified based on the expression of Thy-1, a T- cell related marker. Thy-1 expression allows for the recovery of LTC- ICs from UCB, BM and human fetal liver mononuclear cells (MNCs) and accounts for all repopulating cells (Thy-1.11O) present in mouse BM.

Another marker, CD133 (AC133), a transmembrane receptor glycoprotein has also been shown to coincide with the enrichment of early hematopoietic progenitors.

CD34+CD133+ cell fractions isolated from UCB are highly enriched in primitive progenitors and SRCs that additionally have the capacity to engraft secondary recipients. Recently, vascular growth factor receptor 2 (KDR) has been implicated as a marker for primitive cell types. Studies have shown that the isolation of BM derived CD34+KDR+ results in an enrichment of human LTC-ICs and SRCs.

The absence of specific antigens can also be used to characterize and isolate primitive hematopoietic stem cell populations. For example, human CD34+ cells lacking HLA-DR 36 or CD45RA/CD71 identify primitive multipotential hematopoietic cells capable of self-renewal and differentiation into multiple hematopoietic lineages. Additionally, isolating cells that lack markers associated with mature myeloid and lymphoid cells represents a method of enriching for primitive cell types.

As used herein, the term "differentiated hematopoietic cell", "differentiated cell", or "progenitor cell" refers to a lineage committed hematopoietic cell. These cells typically express one or more of the antigens CD2, CD3, CD14, CD16, CD19, CD24, CD56, CD66b, and glycophorin A, and are termed lineage markers (lin+). The detection of lin+ antigens indicates the loss of pluripotential properties and that the cell has become differentiated, or lineage committed. Accordingly, these fin+ antigens also provide the appropriate antigens for targeted separation of differentiated cells as described herein, and antibodies to these antigens are widely available for immunoseparation procedures.

As described, the majority of culture conditions investigated to date result in the dominance of differentiated cell types and the concomitant decrease in the frequency and numbers of primitive cells, eventually resulting in culture extinction.

This undesired end result is a consequence of several competing factors that can influence culture dynamics. One such parameter is the effect of the endogenous secretion of regulatory molecules, which can be stimulatory or inhibitory to HSC proliferation, by different subpopulations of hematopoietic cells in culture. Using gene expression and protein secretion analysis, a variety of factors known to inhibit HSC expansion were shown to be expressed and secreted by both progenitor and mature cell types-3. For example, monocytes are known to secrete transforming growth factor (TGF)-1 and macrophage inflammatory protein (MIP).

Neutrophils have been associated with the secretion of TGF-, B1, MIP and tumor necrosis factor (TNF) while megakaryocytes secrete interleukin (IL), Similar findings have been documented for erythroid and megakaryocytic progenitors which have been shown to secrete TGF-Q. Furthermore, research has shown that a number of these secreted factors can stimulate the secondary secretion of inhibitory factors by other cell types. For example, the production of IL-12, TNF-a, IL-1, or IL by monocytes can stimulate lymphocytes to produce MIP. These inhibitory factors are known to prevent HSC expansion in vitro by causing them to remain quiescent, undergo apoptosis, and/or differentiate into mature cell types.

Further evidence showing that differentiated cells may inhibit stem cell growth comes from mouse transplant studies where it has been shown that the in vivo expansion potential of mouse repopulating stem cells can actually be limited by the transplantation of increased numbers of stem cells. These reports suggested that the recovery and production of mature blood cells in recipient mice, which arise from the injected repopulating cells, may be responsible for activating inhibitory mechanisms which ultimately limit stem cell proliferation. Accordingly, undifferentiated cells are segregated from these growth factors in culture, which is accomplished by for example but not limited to, media dilution, media exchange, perfusion, and the like, with the object being to reduce local concentrations of growth factors in the culture media.

A demonstration that endogenously secreted factors can negatively influence culture output, comes from studies in which blocking antibodies or agonists (i.e. oligonucleotides or competitive receptor blockers) specifically directed against individual inhibitory factors have been successful in reversing or preventing the effects of known inhibitors such as TGF-,BI, MIP-loc, (MCP)-1 and SDF-1 in both in vitro and in viva models. Unfortunately, the use of such blocking schemes has not propagated into a higher expansion of repopulating HSCs, perhaps because multiple secreted factors are responsible for inhibiting this population. One model for stem cell expansion involves a negative feedback control mechanism whereby differentiated blood cells, generated in cytokine supplemented cultures, produce soluble factors that, directly or indirectly, prevent HSC expansion.

This mechanism implies that the removal of these cells or the endogenous factors generated by these cells would remove the block to HSC expansion by shifting the balance of signals presented to the stem cells (i.e. from supplemented cytokines and secreted cytokines) from those preventing expansion to those favoring expansion.

The removal of these cells may also provide a mechanism to enrich for cells that may secrete stimulatory factors. Usable methods that control and modulate the endogenous production of stimulatory and inhibitory factors thus overcome limitations of current HSC expansion systems.

The removal of specific target cells from culture, coupled with media exchange, results in the concomitant decrease in the endogenous production and overall concentration of inhibitory factors present in culture, which, in turn, results in greater expansion of the HSC populations.

The HSCs generated as described herein can be used for a variety of clinical applications. For example, the expanded HSCs can be transplanted for amelioration of cytopenia and anemia induced by radiotherapy or chemotherapy using anticancer drugs, in order to enhance or accelerate immune and hematopoietic recovery following intensive treatment. Alternatively, the invention can be used for prevention and treatment of infectious diseases associated with lymphopenia, such as the CD4+ T cell depletion seen with chronic HIV infection. The HSCs can be cultured with differentiating factors to produce specific blood cell types. For example, HSCs produced using this invention can be induced to differentiate into cells of a desired population and function using known biological agents. In this manner, the invention can generate "designer transplants" with a plurality of functions established to provide the greatest patient care. The HSCs can also be used in gene therapy, to express a transgene in a recipient subject, taking advantage of their reduced immunogenicity and pluripotential properties.

The present invention provides for expanding stem or progenitor cells, particularly of the hematopoietic lineage. The process generally includes obtaining hematopoietic cells that are enriched for hematopoietic stem and progenitor cells, and introducing them into a suitable growth medium. The cells are maintained in culture and allowed to proliferate. Differentiated cells and endogenous growth factors are removed, either continuously during culture or intermittently during the culture process, for example, through performing media exchange on the cells remaining in culture, and by targeted separation and removal of differentiated cells. The remaining undifferentiated stem cells are cultured and allowed to proliferate further. Multiple cycles of culture and selection/media exchange are performed to expand the cells. Alternatively, differentiated cells in various phases of lineage commitment can be selected and propagated further in accordance with the invention. Likewise, one or more hematopoietins can be added to the culture to force differentiation or lineage commitment. It is preferred that cell expansion and selection be performed in a completely controllable, environmentally closed-system, in accordance with FDA and other regulations governing the handling and processing of blood products, and to maintain sterility.

The bioprocess disclosed herein can be practiced as an open system or as a closed system. Closed systems are generally sealed from the environment, and provide a more regulatable sterile microenvironment for the culture. Additional benefits to closed systems include increased safety for researchers and medical professionals in the handling of biological fluids. Current FDA and other administrative guidelines require closed systems for the handling and processing of blood cells and products designed to be used in humans, and are accordingly preferred. However, open systems exist for the expansion of hematopoietic stem cells, such as those disclosed herein, and for example US patents 5,674,750 and 5,925,567 (each incorporated herein by reference in their entirety) and other known systems can be modified in accordance with the teachings provided herein to produce a suitable open system bioprocess.

The invention consists of one or more cell culture chambers, capable of receiving and containing a sample of cells. The cell culture chambers may be substantially rigid, for example, as in the case of a cell culture flask or dish, or may be semi-rigid, for example, as in the case of a cell culture bag. There are many types and kinds of cell culture containers (chambers) that are commercially available, such as those produced by Corning Costar. Suitable materials are ones that can withstand a variety of sterilization techniques including autoclaving and gamma irradiation and, for those components which directly contact cells, should also be biologically inert. Selection of an appropriate cell culture chamber is made in view of these and such other factors as the volume desired, transparency, gas diffusion, open or closed design, and the particular selection of the type and kind of chamber would be apparent to one of skill in the art in view of the teachings provided. A currently preferred embodiment employs cell culture bags that are semi-permeable to oxygen gas and carbon dioxide gas, but substantially impermeable to water vapor and liquids such as cell culture media, thus ensuring no or little loss of growth medium during culture. Fluorinated ethylene polymers exemplify material suitable for this purpose. Other materials that are not gas permeable but meet the appropriate criteria include polypropylene, stainless steel and other medical grade materials, particularly polymers.

The cell culture chambers may include one or more ports, replaceable caps or covers, self-sealing septa such as rubber stoppers, valves, or similar means that allow the user to add or remove materials from the chamber without substantial exposure of the interior of the bioprocess to the external environment. For example, these mechanisms permit the cells, media and other components, such as antibodies and growth factors, to be introduced into the chamber, and permit removal of media, cells, endogenous soluble growth factors and the like, from the chamber, while maintaining an environmentally closed system. Vents, regulators or other ports for attaching external gas (e.g., oxygen or air) or liquid (e.g., culture media) sources, or for attaching pumps or pressure devices, may be provided.

Under most conditions, ex vivo HSC cultures will attempt to recapitulate hematopoiesis and as such will eventually form a heterogeneous population of cells containing components of the hematopoietic system. Insight into the overall developmental potential and primitiveness of these cells would provide information about the ability of a specific culture methodology to expand primitive cell types.

Developing this knowledge requires robust and quantitative monitoring of cells that are at different stages of differentiation. Various assays have been developed which identify these cells based on distinct functional properties. Cell function can be queried in vitro using established retrospective assays that detect the presence of committed and multipotent progenitor cells based on the formation of morphologically distinguishable colonies. Colony forming cells (CFCs) are progenitor cells that can be detected by the formation of erythroid, myeloid or mixed (i.e. both erythroid and myeloid) cell containing colonies after 2-3 weeks of culture in semi-solid media (methycellulose). Long-term culture-initiating cells (LTC-ICs) are more primitive than CFCs and can be enumerated by their ability to give rise to CFCs after greater than 5-weeks of culture with stromal cells. The stromal cell elements, which are composed of mesenchymal cells including fibroblasts, endothelial cells, adipocytes and osteogenic cells, produce a variety of soluble factors that support the long-term proliferation and maintenance of LTC-ICs. The sensitivity of this assay can be increased through the use of genetically engineered murine fibroblast (M2-1OB4) cell lines that secrete factors known to enhance the detection I and maintenance of LTC-ICs.

In vivo functional assays offer the best indication of the developmental potential of a hematopoietic cell population. This is because they directly test the potential for a stem cell population to contribute to the development or re development of a particular organ, tissue or system following intravenous injection. For example, murine HSCs have been identified based on their ability to reconstitute hematopoiesis after transplantation into an immunocompromised and hematologically compromised host. Till and McCulloch first reported the existence of such a cell type when they injected syngeneic BM cells into irradiated mouse recipients and observed the formation of multi-lineage colonies in the spleen.

Hematopoietins are a generic name given to hematopoietic growth factors (HGF) or hematopoietic cytokines, which act on cells of the hematopoietic system. These factors are active at all stages of development, and accordingly these hematopoietins will be removed from the bioprocess to prevent HSC differentiation.

Hematopoietic growth factors are produced by many different cell types including those not belonging to the hematopoietic system. These factors are either secreted or they exist in membrane-bound or matrix-associated forms. They may have different modes of action also, such as autocrine, paracrine, or juxtacrine growth control. Production of hematopoietic factors is regulated strictly, i.e., they are synthesized by activated cells under certain conditions rather than being produced constitutively all the time. Many observations point to the existence of an ordered hierarchy and a concerted action of factors involved in the development of the hematopoietic system. These factors are required for the maintenance of hematopoietic stem cells, their proliferation, their differentiation into different hematopoietic lineages, and for the maintenance of a stable equilibrium between proliferation and differentiation. These factors allow an organism to shift this equilibrium to one or the other side, as required, for example, under stress conditions. Many of these factors overlap in their biological activities. Teleologically this guarantees a high efficiency and also allows substitution and/or complementation of individual components the functions of which may have been impaired, for example, under pathological conditions. In addition, responses elicited by these factors are usually contextual, i.e. these responses depend on the presence and concentration of other cytokines and/or factors in the environment of the responding cells. The majority of studies aimed at stimulating HSC expansion in vitro focus on the use of exogenous cytokine supplementation strategies. Cytokines interact with HSCs via three classes of transmembrane receptors; 1) those with intrinsic tvrosine kinase activity, 2) those that interact with the gpl30 subunit and 3) those that interact with Janus kineses (JAKs). Over the years, the use of phenotypic and functional assays has identified a number of cytokines which have distinct stimulatory effects on primitive hematopoietic cells. These include flk2/flt3 ligand (FL), stem cell factor (SCF), interleukin (IL)-6, IL-6/soluble IL-6-receptor (SIL-6R), IL- 11, thrombopoietin (TPO), IL-3, IL-1, IL-12, granulocyte- colony stimulating factor (G-CSF) and granulocyte- macrophage- colony stimulating factor (GM-CSF) 90-0. The first reported use of stroma-free cytokine supplemented cultures, which contained IL-1, IL-3, IL-6, G-CSF, GM-CSF and SCF, supported a significant expansion (66-fold) of colony forming unit- granulocyte-macrophage (CFU-GM) progenitor cells.

Exemplary culture conditions for growing HSCs are given in the Examples, but generally in accordance with the invention, a sample of cells containing a subset of HSCs is first obtained then cultured. The cultured cells are then maintained for a growth period suitable for allowing proliferation to occur, which may include media exchanges to remove soluble growth factors, after which time the HSCs are segregated from the other differentiated cells. The HSCs are then allowed to proliferate again as described. The segregation of differentiated cells can be performed again if necessary. At the end of the culture period the expanded HSCs can be preserved by freezing after addition of, for example glycerin, DMSO or a suitable cryopreservative, or used directly in a therapeutic procedure. It is important to note that the above steps can be performed with the entire bioprocess apparatus assembled or in separate parts in which cell culture is carried out independent of cell segregation.

Clinical uses for HSCs include, for example, the therapeutic treatment of blood cancers treatment of anemia, treatment of hereditary blood disorders, replenishment of blood cells following high dose radiation and chemotherapy in the treatment of cancer, graft-versus-tumor treatment of cancer, treatment of autoimmune disorders, and in gene therapy approaches.

For the therapeutic treatment of blood cancers, including lymphoblastic leukemia, acute myeloblastic leukemia, chronic myelogenous leukemia (CML), Hodgkin's disease, multiple myeloma, and non-Hodgkin's and B-cell lymphomas, a patient's own cancerous hematopoietic cells are first destroyed by high dose radiation and chemotherapy. The patient provides the source of transplantable HSCs, which are isolated and expanded according to the methods provided herein. The transplant of undifferentiated cells provides for long term repopulation of the blood of the recipient. Non-cancerous blood disorders amenable to treatment by HSC therapy include aplastic and other types of anemia. The transplant of undifferentiated cells provides for long term repopulation of the blood of the recipient. Using UCB, multiple studies have; demonstrated that cell dose is an important determinant of patient survival in stem cell transplantation scenarios.

The invention is now described in specific terms by the foregoing examples, which are illustrative only and are intended to be non-limiting and specific embodiments, whereas the full scope of the invention shall be determined solely by the claims.

### EXAMPLES

### Example 1

### Effect of X (Human bone marrow extracellular matrix extract) on Engraftment of NOD-SCID Mice with 3M CB MNC's after 7 days in culture

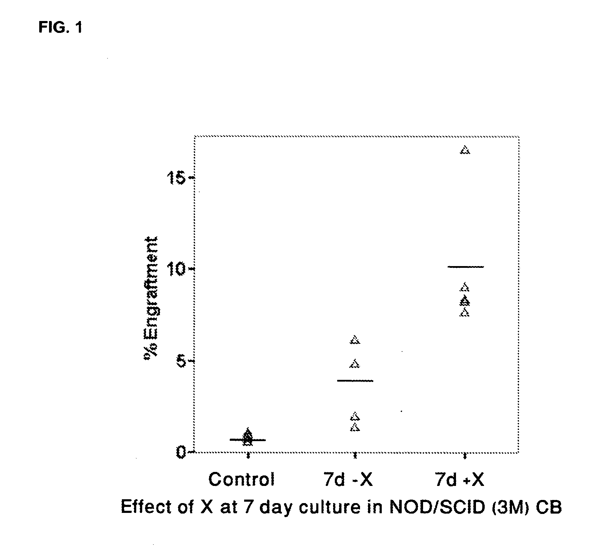

A control group of 5 mice were ablated by Busilvex but did not receive any IV injection of CB MNCs. Two groups of 5 mice each received 3M CB MNCs that were put in culture for 7 days in the presence or absence of X. The % of engraftment increased from 3% in the mice without X to more than 10% in the mice with X. As appears from FIG. 1 the difference was statistically significant between the 2 groups of mice (P < 0.05). The increase in engraftment is also shown with the human pan leukocytic (CD45), B lymphocytes (CD19) and myeloid cells (CD33) markers.

### Example 2

### Effect of X (Human bone marrow extracellular matrix extract) on Engraftment of NOG Mice with CB MNC after 7 days in culture

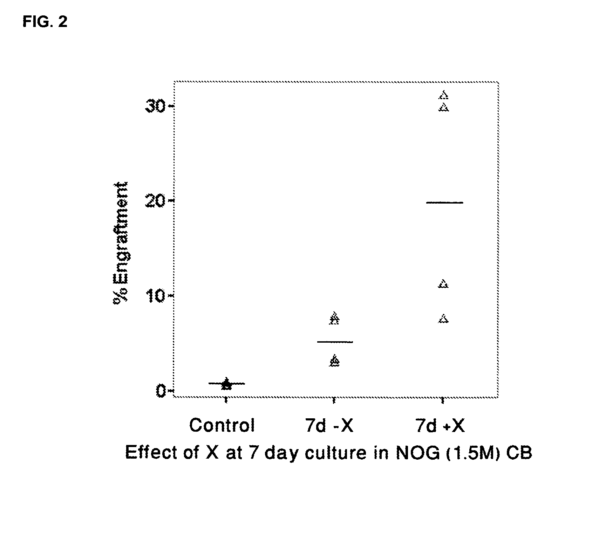

A control group of 4 mice were ablated by Busilvex but did not receive any IV injection of CB MNCs. Two groups of 4 mice each received 1.5M CB MNCs that were put in culture for 7 days in the presence or absence of X. The % of engraftment increased from 5% in the mice without X to 20% in the mice with X. As appears from FIG. 2 the difference was statistically significant between the 2 groups (P < 0.05). The use of a linear model based on the negative binomial distribution yields even a higher statistically significant difference between the two groups. The increase in engraftment is also shown with the human pan leukocytic (CD45), B lymphocytes (CD19) and myeloid cells (CD33) markers.

### Example 3

### Engraftment of NOG mice with 4M CB MNC's +/-X (Human bone marrow extracellular matrix extract) for 7days, assessed at 3 weeks

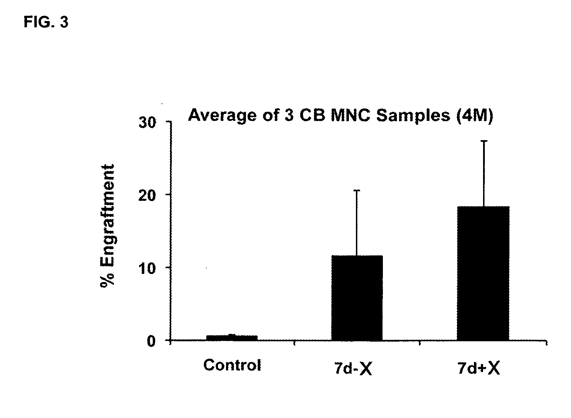

A control group of 3 mice were ablated by Busilvex but did not receive any IV injection of CB MNCs. Two groups of 3 mice each received 4M CB MNCs that were put in culture for 7 days in the presence or absence of X. The % of engraftment was assessed at 3 weeks, instead of 6 weeks, in order to assess immature erythroblasts and erythroid differentiation. As appears from FIG. 3 the engraftment increased from 11% in the mice without X to 18% in the mice with X. The increase in engraftment was due mainly to increase in the numbers of immature erythroblasts (CD45-CD36+, heavy gray).

## Claims

1. Cellular preparation for use in autologous hematopoietic cell transplantation in a patient by administering the cellular preparation to the patient for the purpose of reconstituting both the defence and long-term engraftment,
wherein said cellular preparation is obtainable by a method involving:
i) removing hematopoietic stem cells from the patient;
ii) optionally expanding the hematopoietic stem cells ex vivo with a growth factor selected from the group consisting of GM-CSF, SF, IL-3, IL- 1, GM-CSF/IL-3 fusion proteins, and combinations thereof to provide a cellular preparation comprising an expanded population of hematopoietic progenitor cells;
iii) contacting a fraction of the hematopoietic stem cells with a human bone marrow extracellular matrix extract and soluble factors to differentiate the cells into a myeloid cell lineage;
iv) combining the hematopoietic stem cells and the myeloid cell lineage to form the cellular preparation.

2. The cellular preparation for use according to claim 1 in which the patient undergoes or has undergone treatment with a cytoreduction agent.

3. The cellular preparation for use according to claim 1 in which the patient suffers from a hematopoietic malignancy.

4. The cellular preparation for use according to claim 1 in which the patient suffers from anemia.

5. The cellular preparation for use according to claim 1 in which the cellular composition is administered intravenously.

6. Cellular preparation for use in reconstituting both the defence and long-term engraftment in a patient with a blood cancer said preparation obtainable by a method involving:
i) removing hematopoietic stem cells from the patient;
ii) optionally expanding the hematopoietic stem cells ex vivo with a growth factor selected from the group consisting of GM-CSF, SF, IL-3, IL- 1, GM-CSF/IL-3 fusion proteins, and combinations thereof to provide a cellular preparation comprising an expanded population of hematopoietic progenitor cells;
iii) contacting a fraction of the hematopoietic stem cells with a human bone marrow extracellular matrix extract and soluble factors to differentiate the cells into a myeloid cell lineage;
iv) combining the hematopoietic stem cells and the myeloid cell lineage to form the cellular preparation.

## Patentansprüche

1. Zelluläres Präparat zur Anwendung in der autologen hämatopoetischen Zell-Transplantation durch Verabreichung des zellulären Präparats an den Patienten, für den Zweck der Rekonstitution sowohl der Abwehr als auch der permanenten Hämatopoese *(long-term engraftment),*
wobei das zelluläre Präparat durch ein Verfahren erhältlich ist, beinhaltend:
i. Entnehmen hämatopoetischer Stammzellen von dem Patienten;
ii. Eventuell Anreichern der hämatopoetischen Stammzellen ex vivo mit einem Wachstumsfaktor, ausgewählt aus der Gruppe bestehend aus GM-CSF, SF, IL-3, IL-1, GM-CSF/IL-3 Fusionsproteinen, und Kombinationen daraus, um ein zelluläres Präparat bereitzustellen, das einen erweiterten Bestand an hämatopoetischen Vorläuferzellen umfasst;
iii. Inkontaktbringen einer Fraktion der hämatopoetischen Stammzellen mit einem Extrakt der extrazellulären Matrix von menschlichem Knochenmark und löslichen Faktoren, um die Zellen in eine myeloide Zelllinie zu differenzieren;
iv. Kombinieren der hämatopoetischen Stammzellen und der myeloiden Zelllinie zur Bildung des zellulären Präparats.

2. Zelluläres Präparat zur Anwendung gemäß Anspruch 1, wobei der Patient einer Behandlung mit einem Mittel zur Zytoreduktion unterzogen wird oder unterzogen wurde.

3. Zelluläres Präparat zur Anwendung gemäß Anspruch 1, wobei der Patient an einer malignen hämatologischen Erkrankung leidet.

4. Zelluläres Präparat zur Anwendung gemäß Anspruch 1, wobei der Patient an Anämie leidet.

5. Zelluläres Präparat zur Anwendung gemäß Anspruch 1, wobei die zelluläre Zusammensetzung intravenös verabreicht wird.

6. Zelluläres Präparat zur Anwendung bei der Rekonstitution sowohl der Abwehr als auch der permanenten Hämatopoese (long-term engraftment) bei einem Patienten mit Blutkrebs, wobei das Präparat erhältlich ist durch ein Verfahren, beinhaltend:
i. Entnehmen hämatopoetischer Stammzellen von dem Patienten;
ii. Eventuell Anreichern der hämatopoetischen Stammzellen ex vivo mit einem Wachstumsfaktor, ausgewählt aus der Gruppe bestehend aus GM-CSF, SF, IL-3, IL-1, GM-CSF/IL-3 Fusionsproteinen, und Kombinationen daraus, um ein zelluläres Präparat bereitzustellen, das einen erweiterten Bestand an hämatopoetischen Vorläuferzellen umfasst;
iii. Inkontaktbringen einer Fraktion der hämatopoetischen Stammzellen mit einem Extrakt der extrazellulären Matrix von menschlichem Knochenmark und löslichen Faktoren, um die Zellen in eine myeloide Zelllinie zu differenzieren;
iv. Kombinieren der hämatopoetischen Stammzellen und der myeloiden Zelllinie zur Bildung des zellulären Präparats.

## Revendications

1. Préparation cellulaire pour l'utilisation dans une greffe autologue de cellules hématopoïétiques chez un patient en administrant la préparation cellulaire au patient pour la reconstitution à la fois la défense et la prise de greffe à long terme, dans laquelle la préparation cellulaire pouvant être obtenue par une méthode comprenant .
i) l'élimination des cellules souches hématopoïétiques à partir du patient;
ii) éventuellement l'expansion des cellules souches hématopoïétiques ex vivo avec un facteur de croissance choisi du groupe consistant en GM-CSF, le SF, IL-3, IL-1, les protéines de fusion GM-CSF/IL-3, et des combinaisons de ceux-ci pour fournir un préparation cellulaire comprenant une population étendue de cellules souches hématopoïétiques;
iii) la mise en contact d'une fraction des cellules souches hématopoïétiques avec un extrait de matrice extracellulaire de la moelle osseuse humaine et des facteurs solubles, pour différencier les cellules dans une lignée cellulaire myéloïde;
iv) la combinaison des cellules souches hématopoïétiques et de cellules de la lignée myéloïde pour former une préparation cellulaire.

2. Préparation cellulaire pour l'utilisation selon la revendication 1, dans laquelle le patient subit ou a subi un traitement avec un agent de cytoréduction.

3. Préparation cellulaire pour l'utilisation selon la revendication 1, dans laquelle le patient souffre d'une malignité hématopoïétique.

4. Préparation cellulaire pour l'utilisation selon la revendication 1, dans laquelle le patient souffre d'une anémie.

5. Préparation cellulaire pour l'utilisation selon la revendication 1, dans laquelle la composition cellulaire est administré par voie intraveineuse.

6. Préparation cellulaire pour l'utilisation pour la reconstitution à la fois la défense et la prise de greffe à long terme dans un patient ayant du cancer du sang, ladite préparation pouvant être obtenue par une méthode comprenant .
i) l'élimination des cellules souches hématopoïétiques à partir du patient;
ii) éventuellement l'expansion des cellules souches hématopoïétiques ex vivo avec un facteur de croissance choisi du groupe consistant en GM-CSF, le SF, IL-3, IL-1, les protéines de fusion GM-CSF/IL-3, et des combinaisons de ceux-ci pour fournir un préparation cellulaire comprenant une population étendue de cellules souches hématopoïétiques;
iii) la mise en contact d'une fraction des cellules souches hématopoïétiques avec un extrait de matrice extracellulaire de la moelle osseuse humaine et des facteurs solubles, pour différencier les cellules dans une lignée cellulaire myéloïde;
iv) la combinaison des cellules souches hématopoïétiques et de cellules de la lignée myéloïde pour former une préparation cellulaire.
